# EUROPEAN PATENT APPLICATION

(11) **EP 4 830 988 A1**
(43) Date of publication of application: **28.10.2026**
(21) Application number: 26172414.0
(22) Date of filing: 15.04.2026
(51) Int. Cl.: B60N 2/02, A61B 5/18, B60N 2/00, B60N 2/90

(54) **METHOD AND SYSTEM FOR TUNING VIBRO SEAT**

(30) Priority: 21.04.2025 KR 20250051773
(71) Applicant: Hyundai Mobis Co., Ltd., Gangnam-gu Seoul 06141 (KR)
(72) Inventor: LIM, Hyung Sub, Yongin-si (KR); BAEK, Ji Seon, Yongin-si (KR)
(74) Representative: Vossius & Partner Patentanwälte Rechtsanwälte mbB

(57) **Abstract**

Described is a method for tuning a vibro seat, the method comprising: generating vehicle customer relationship management (VCRM) data based on biometric information of an occupant and music preference information of the occupant; generating a vibro seat tuning parameter based on the generated VCRM data; adjusting and tuning an envelope function based on the occupant biometric information and the occupant music preference information received in real time and the vibro seat tuning parameter (S230, S240); and operating the vibro seat based on the tuned envelope function (S250).

## Description

### BACKGROUND OF THE DISCLOSURE

### Field of the Disclosure

The present embodiments relate to a vibro seat, and more particularly, to a vehicle to which a vibro seat tuning method is applied.

### Discussion of the Related Art

As standards of living improve, the use of personal leisure time and convenience of life have increased, and automobile culture has rapidly changed under such influences. In recent years, the time spent in vehicles has increased, and thus vehicles have become cultural media serving as personal living spaces beyond simple means of transportation.

As the time spent in vehicles increases as such, the physical burden on occupants in a vehicle increases. Particularly, vibrations transmitted from the road surface are transmitted to the human body through a vehicle body. Vibrations generated when traveling in an area where the road surface is in a bad condition may cause damage to the body or nervous system, and thus symptoms such as fatigue, headache, low back pain, and decreased vision may occur.

A seat suspension is used to solve the above problems, that is, to support an occupant while maintaining maximum riding comfort of a human on a seat. Such a seat suspension is intended to minimize and absorb impacts or shaking generated when traveling on uneven roads, thereby improving the riding comfort of the occupant.

Recently, there has been an increasing need for a more improved music playback environment during vehicle operation. One example of such need may be a vibration seat. The vibration seat is an apparatus capable of increasing satisfaction of a vehicle occupant during audio playback by mounting a vibration generating means in a vehicle seat and generating vibrations corresponding to the audio currently being played.

However, a general vibration seat does not reflect the physiological condition or music preference of an occupant, and thus a user may feel a sense of incongruity.

### SUMMARY OF THE DISCLOSURE

In order to solve the above-described problems, an embodiment of the present disclosure is intended to provide a method and a system for tuning a vibro seat that provide a vibro seat operation customized for an occupant by reflecting biometric information and music preference of the occupant.

Technical purposes to be achieved by the present disclosure are not limited to the technical purposes mentioned above, and other technical purposes not mentioned will be clearly understood by those skilled in the art to which the present disclosure pertains from the following description.

Provided is a method for tuning a vibro seat according to one of embodiments of the present disclosure for achieving the above-described technical purposes, the method comprising: generating vehicle customer relationship management (VCRM) data based on biometric information of an occupant and music preference information of the occupant; generating a vibro seat tuning parameter based on the generated VCRM data; adjusting and tuning an envelope function based on the occupant biometric information and the occupant music preference information received in real time and the vibro seat tuning parameter; and operating the vibro seat based on the tuned envelope function.

According to an embodiment, the generating of the VCRM data may include: determining a physical condition based on the occupant biometric information; generating a physical condition parameter based on the determined physical condition; determining a mental preference based on the occupant music preference information; generating a mental preference parameter based on the determined mental preference; and generating the vibro seat tuning parameter by matching the physical condition parameter and the mental preference parameter with each other.

According to an embodiment, the determining of the physical condition based on the occupant biometric information may include: determining a facial expression-based tension level by comparing facial image information of the occupant with a facial expression-based determination logic; determining a pulse/heart rate-based tension level by comparing pulse information and heart rate information of the occupant with a pulse/heart rate-based determination logic; and determining the physical condition by performing a weighted summation of the facial expression-based tension level and the pulse/heart rate-based tension level.

According to an embodiment, the generating of the physical condition parameter based on the determined physical condition may include: generating a first band pass filter parameter; generating a first compressor parameter; and generating a first output gain parameter.

According to an embodiment, the determining of the mental preference based on the occupant music preference information may include: collecting new playback information of the occupant accumulated within a vehicle; collecting pre-stored previous playback information of the occupant; and determining the mental preference by comparing the new playback information and the previous playback information with a music preference determination logic.

According to an embodiment, the generating of the mental preference parameter based on the determined mental preference may include: generating a second band pass filter parameter; generating a second compressor parameter; and generating a second output gain parameter.

According to an embodiment, the generating of the vibro seat tuning parameter by matching the physical condition parameter and the mental preference parameter with each other may include: generating a third band pass filter parameter by performing a weighted summation of the first band pass filter parameter and the second band pass filter parameter; generating a third compressor parameter by performing a weighted summation of the first compressor parameter and the second compressor parameter; and generating a third output gain filter parameter by performing a weighted summation of the first output gain parameter and the second output gain parameter.

According to an embodiment, the adjusting and tuning of the envelope function may include: receiving audio output in real time within the vehicle; applying a band pass filter configured with the third band pass filter parameter to the received audio; applying a compressor configured with the third compressor parameter to the audio to which the band pass filter has been applied; extracting an envelope by applying a low pass filter to the audio to which the compressor has been applied; generating a sinusoidal signal; and generating a vibro seat signal by performing a convolution on the extracted envelope and the generated sinusoidal signal.

According to an embodiment, the adjusting and tuning of the envelope function may further include: receiving vehicle travel data including driving mode information and speed information; adjusting the vibro seat tuning parameter based on the driving mode information; and adjusting an output gain weight based on the speed information.

According to one of the embodiments of the present disclosure, safety and ride comfort may be improved during travel through a vibro seat operation customized for an occupant.

Effects that may be obtained in the present disclosure are not limited to the effects mentioned above, and other effects not mentioned will be clearly understood by those skilled in the art to which the present disclosure pertains from the following description.

### BRIEF DESCRIPTION OF THE DRAWINGS

FIG. 1 is an overall block diagram of an autonomous driving control system to which an autonomous driving apparatus according to any one of embodiments of the present disclosure is applicable.
FIG. 2 is a diagram illustrating an example in which an autonomous driving apparatus according to any one of embodiments of the present disclosure is applied to a vehicle.
FIGS. 3 to 8 are diagrams for describing a vibro seat tuning system according to embodiments of the present disclosure.
FIG. 9 is a diagram for describing a method for generating a vibro seat signal according to embodiments of the present disclosure.
FIGS. 10 and 11 are flowcharts for describing a method for tuning a vibro seat according to embodiments of the present disclosure.

### DETAILED DESCRIPTION OF THE DISCLOSURE

Hereinafter, with reference to the accompanying drawings, embodiments of the present disclosure will be described in detail so that those skilled in the art can easily practice the present disclosure. However, the present disclosure may be implemented in various different forms and is not limited to the embodiments described herein. In addition, in the drawings, in order to clearly explain the present disclosure, parts that are not related to the description will be omitted, and the same or similar parts are denoted by the same reference numerals throughout the description.

Throughout the description, when a part is referred to as "including" an element, it may not mean that the part excludes other elements, but may mean that the part includes other elements, unless stated otherwise.

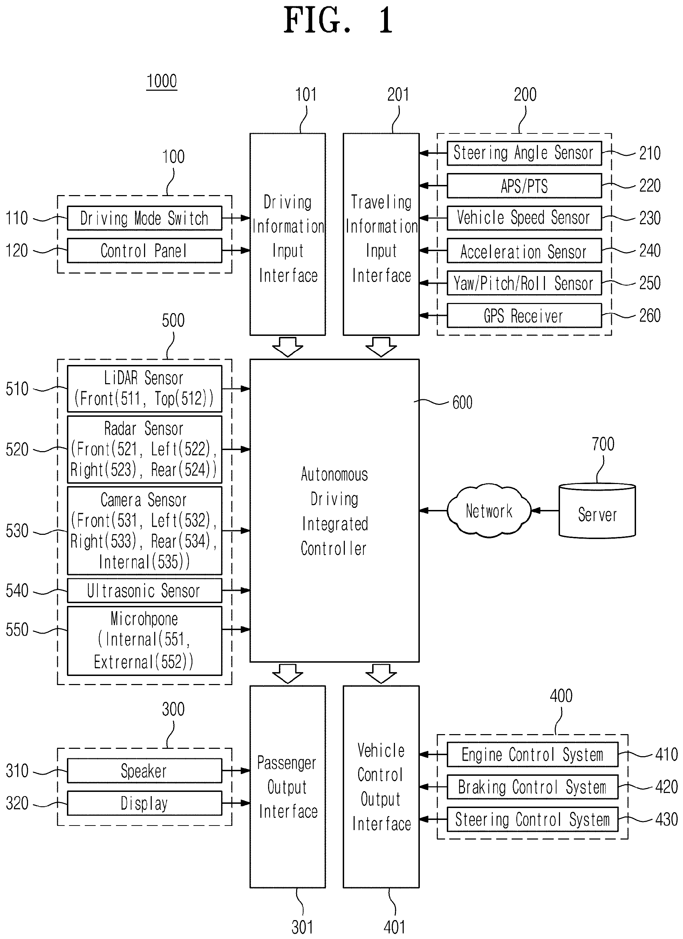

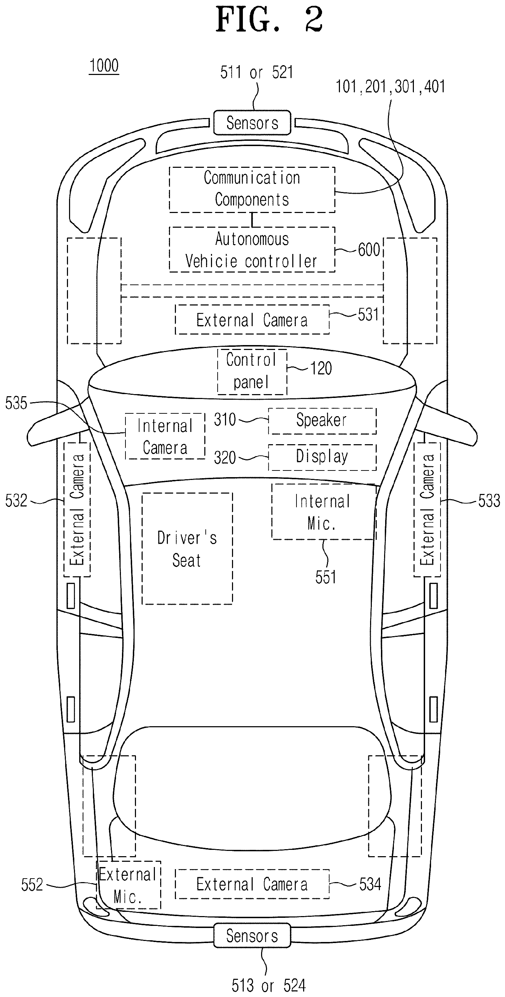

FIG. 1 is an overall block diagram of an autonomous driving control system to which an autonomous driving apparatus according to any one of embodiments of the present disclosure is applicable. FIG. 2 is a diagram illustrating an example in which an autonomous driving apparatus according to any one of embodiments of the present disclosure is applied to a vehicle.

First, a structure and function of an autonomous driving control system (e.g., an autonomous driving vehicle) to which an autonomous driving apparatus according to the present embodiments is applicable will be described with reference to FIGS. 1 and 2.

As illustrated in FIG. 1, an autonomous driving vehicle 1000 may be implemented based on an autonomous driving integrated controller 600 that transmits and receives data necessary for autonomous driving control of a vehicle through a driving information input interface 101, a traveling information input interface 201, an occupant output interface 301, and a vehicle control output interface 401. However, the autonomous driving integrated controller 600 may also be referred to herein as a controller, a processor, or, simply, a controller.

The autonomous driving integrated controller 600 may obtain, through the driving information input interface 101, driving information based on manipulation of an occupant for a user input unit 100 in an autonomous driving mode or manual driving mode of a vehicle. As illustrated in FIG. 1, the user input unit 100 may include a driving mode switch 110 and a control panel 120 (e.g., a navigation terminal mounted on the vehicle or a smartphone or tablet computer owned by the occupant). Accordingly, driving information may include driving mode information and navigation information of a vehicle.

For example, a driving mode (i.e., an autonomous driving mode/manual driving mode or a sports mode/eco mode/safety mode/normal mode) of the vehicle determined by manipulation of the occupant for the driving mode switch 110 may be transmitted to the autonomous driving integrated controller 600 through the driving information input interface 101 as the driving information.

Furthermore, navigation information, such as the destination of the occupant input through the control panel 120 and a path up to the destination (e.g., the shortest path or preference path, selected by the occupant, among candidate paths up to the destination), may be transmitted to the autonomous driving integrated controller 600 through the driving information input interface 101 as the driving information.

The control panel 120 may be implemented as a touchscreen panel that provides a user interface (UI) through which the occupant inputs or modifies information for autonomous driving control of the vehicle. In this case, the driving mode switch 110 may be implemented as touch buttons on the control panel 120.

In addition, the autonomous driving integrated controller 600 may obtain traveling information indicative of a driving state of the vehicle through the traveling information input interface 201. The traveling information may include a steering angle formed when the occupant manipulates a steering wheel, an accelerator pedal stroke or brake pedal stroke formed when the occupant depresses an accelerator pedal or brake pedal, and various types of information indicative of driving states and behaviors of the vehicle, such as a vehicle speed, acceleration, a yaw, a pitch, and a roll formed in the vehicle. The traveling information may be detected by a traveling information detection unit 200, including a steering angle sensor 210, an accelerator position sensor (APS)/pedal travel sensor (PTS) 220, a vehicle speed sensor 230, an acceleration sensor 240, and a yaw/pitch/roll sensor 250, as illustrated in FIG. 1.

Furthermore, the traveling information of the vehicle may include location information of the vehicle. The location information of the vehicle may be obtained through a global positioning system (GPS) receiver 260 applied to the vehicle. Such traveling information may be transmitted to the autonomous driving integrated controller 600 through the traveling information input interface 201 and may be used to control the driving of the vehicle in the autonomous driving mode or manual driving mode of the vehicle.

The autonomous driving integrated controller 600 may transmit driving state information provided to the occupant to an output unit 300 through the occupant output interface 301 in the autonomous driving mode or manual driving mode of the vehicle. That is, the autonomous driving integrated controller 600 transmits the driving state information of the vehicle to the output unit 300 so that the occupant may check the autonomous driving state or manual driving state of the vehicle based on the driving state information output through the output unit 300. The driving state information may include various types of information indicative of driving states of the vehicle, such as a current driving mode, transmission range, and speed of the vehicle.

If it is determined that it is necessary to warn a driver in the autonomous driving mode or manual driving mode of the vehicle along with the above driving state information, the autonomous driving integrated controller 600 transmits warning information to the output unit 300 through the occupant output interface 301 so that the output unit 300 may output a warning to the driver. In order to output such driving state information and warning information acoustically and visually, the output unit 300 may include a speaker 310 and a display 320 as illustrated in FIG. 1. In this case, the display 320 may be implemented as the same device as the control panel 120 or may be implemented as an independent device separated from the control panel 120.

Furthermore, the autonomous driving integrated controller 600 may transmit control information for driving control of the vehicle to a lower control system 400, applied to the vehicle, through the vehicle control output interface 401 in the autonomous driving mode or manual driving mode of the vehicle. As illustrated in FIG. 1, the lower control system 400 for driving control of the vehicle may include an engine control system 410, a braking control system 420, and a steering control system 430. The autonomous driving integrated controller 600 may transmit engine control information, braking control information, and steering control information, as the control information, to the respective lower control systems 410, 420, and 430 through the vehicle control output interface 401. Accordingly, the engine control system 410 may control the speed and acceleration of the vehicle by increasing or decreasing fuel supplied to an engine. The braking control system 420 may control the braking of the vehicle by controlling braking power of the vehicle. The steering control system 430 may control the steering of the vehicle through a steering device (e.g., motor driven power steering (MDPS) system) applied to the vehicle.

As described above, the autonomous driving integrated controller 600 according to the present embodiment may obtain the driving information based on manipulation of the driver and the traveling information indicative of the driving state of the vehicle through the driving information input interface 101 and the traveling information input interface 201, respectively, and transmit the driving state information and the warning information, generated based on an autonomous driving algorithm, to the output unit 300 through the occupant output interface 301. In addition, the autonomous driving integrated controller 600 may transmit the control information generated based on the autonomous driving algorithm to the lower control system 400 through the vehicle control output interface 401 so that driving control of the vehicle is performed.

In order to guarantee stable autonomous driving of the vehicle, it is necessary to continuously monitor the driving state of the vehicle by accurately measuring a driving environment of the vehicle and to control driving based on the measured driving environment. To this end, as illustrated in FIG. 1, the autonomous driving apparatus according to the present embodiment may include a sensor unit 500 for detecting a nearby object of the vehicle, such as a nearby vehicle, pedestrian, road, or fixed facility (e.g., a signal light, a signpost, a traffic sign, or a construction fence).

The sensor unit 500 may include one or more of a LiDAR sensor 510, a radar sensor 520, or a camera sensor 530, in order to detect a nearby object outside the vehicle, as illustrated in FIG. 1.

The LiDAR sensor 510 may transmit a laser signal to the periphery of the vehicle and detect a nearby object outside the vehicle by receiving a signal reflected and returning from a corresponding object. The LiDAR sensor 510 may detect a nearby object located within the ranges of a preset distance, a preset vertical field of view, and a preset horizontal field of view, which are predefined depending on specifications thereof. The LiDAR sensor 510 may include a front LiDAR sensor 511, a top LiDAR sensor 512, and a rear LiDAR sensor 513 installed at the front, top, and rear of the vehicle, respectively, but the installation location of each LiDAR sensor and the number of LiDAR sensors installed are not limited to a specific embodiment. A threshold for determining the validity of a laser signal reflected and returning from a corresponding object may be previously stored in a memory (not illustrated) of the autonomous driving integrated controller 600. The autonomous driving integrated controller 600 may determine a location (including a distance to a corresponding object), speed, and moving direction of the corresponding object using a method of measuring time taken for a laser signal, transmitted through the LiDAR sensor 510, to be reflected and returning from the corresponding object.

The radar sensor 520 may radiate electromagnetic waves around the vehicle and detect a nearby object outside the vehicle by receiving a signal reflected and returning from a corresponding object. The radar sensor 520 may detect a nearby object within the ranges of a preset distance, a preset vertical field of view, and a preset horizontal field of view, which are predefined depending on specifications thereof. The radar sensor 520 may include a front radar sensor 521, a left radar sensor 522, a right radar sensor 523, and a rear radar sensor 524 installed at the front, left, right, and rear of the vehicle, respectively, but the installation location of each radar sensor and the number of radar sensors installed are not limited to a specific embodiment. The autonomous driving integrated controller 600 may determine a location (including a distance to a corresponding object), speed, and moving direction of the corresponding object using a method of analyzing power of electromagnetic waves transmitted and received through the radar sensor 520.

The camera sensor 530 may detect a nearby object outside the vehicle by photographing the periphery of the vehicle and detect a nearby object within the ranges of a preset distance, a preset vertical field of view, and a preset horizontal field of view, which are predefined depending on specifications thereof.

The camera sensor 530 may include a front camera sensor 531, a left camera sensor 532, a right camera sensor 533, and a rear camera sensor 534 installed at the front, left, right, and rear of the vehicle, respectively, but the installation location of each camera sensor and the number of camera sensors installed are not limited to a specific embodiment. The autonomous driving integrated controller 600 may determine a location (including a distance to a corresponding object), speed, and moving direction of the corresponding object by applying predefined image processing to an image captured by the camera sensor 530.

In addition, an internal camera sensor 535 for capturing the inside of the vehicle may be mounted at a predetermined location (e.g., rear view mirror) within the vehicle. The autonomous driving integrated controller 600 may monitor a behavior and state of the occupant based on an image captured by the internal camera sensor 535 and output guidance or a warning to the occupant through the output unit 300.

As illustrated in FIG. 1, the sensor unit 500 may further include an ultrasonic sensor 540 in addition to the LiDAR sensor 510, the radar sensor 520, and the camera sensor 530 and further adopt various types of sensors for detecting a nearby object of the vehicle along with the sensors.

FIG. 2 illustrates an example in which, in order to aid in understanding the present embodiment, the front LiDAR sensor 511 or the front radar sensor 521 is installed at the front of the vehicle, the rear LiDAR sensor 513 or the rear radar sensor 524 is installed at the rear of the vehicle, and the front camera sensor 531, the left camera sensor 532, the right camera sensor 533, and the rear camera sensor 534 are installed at the front, left, right, and rear of the vehicle, respectively. However, as described above, the installation location of each sensor and the number of sensors installed are not limited to a specific embodiment.

Furthermore, in order to determine a state of the occupant within the vehicle, the sensor unit 500 may further include a bio sensor for detecting bio signals (e.g., heart rate, electrocardiogram, respiration, blood pressure, body temperature, electroencephalogram, photoplethysmography (or pulse wave), and blood sugar) of the occupant. The bio sensor may include a heart rate sensor, an electrocardiogram sensor, a respiration sensor, a blood pressure sensor, a body temperature sensor, an electroencephalogram sensor, a photoplethysmography sensor, and a blood sugar sensor.

Finally, the sensor unit 500 additionally includes a microphone 550 having an internal microphone 551 and an external microphone 552 used for different purposes.

The internal microphone 551 may be used, for example, to analyze the voice of the occupant in the autonomous driving vehicle 1000 based on AI or to immediately respond to a direct voice command of the occupant.

In contrast, the external microphone 552 may be used, for example, to appropriately respond to safe driving by analyzing various sounds generated from the outside of the autonomous driving vehicle 1000 using various analysis tools such as deep learning.

For reference, the symbols illustrated in FIG. 2 may perform the same or similar functions as those illustrated in FIG. 1. FIG. 2 illustrates in more detail a relative positional relationship of each component (based on the interior of the autonomous driving vehicle 1000) as compared with FIG. 1.

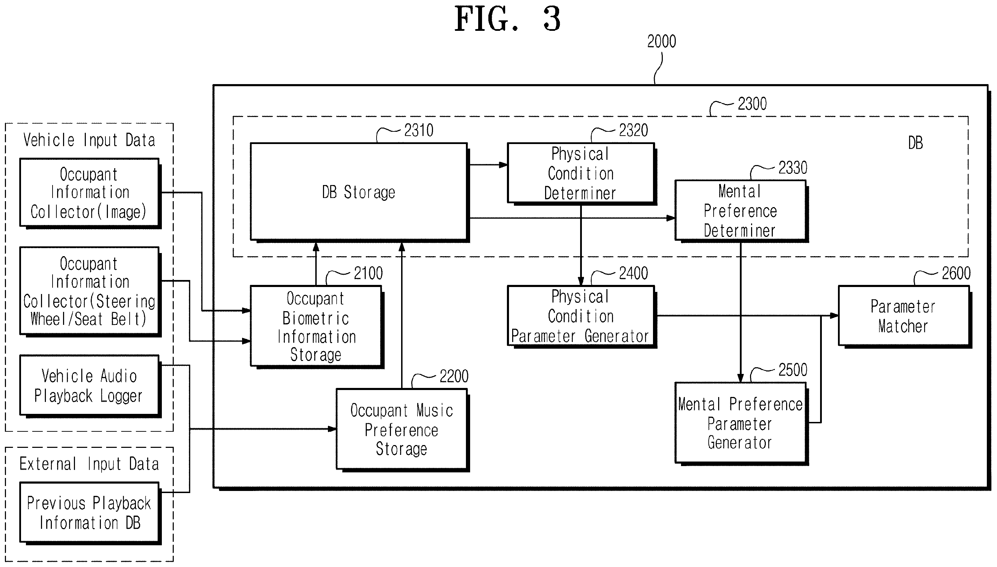

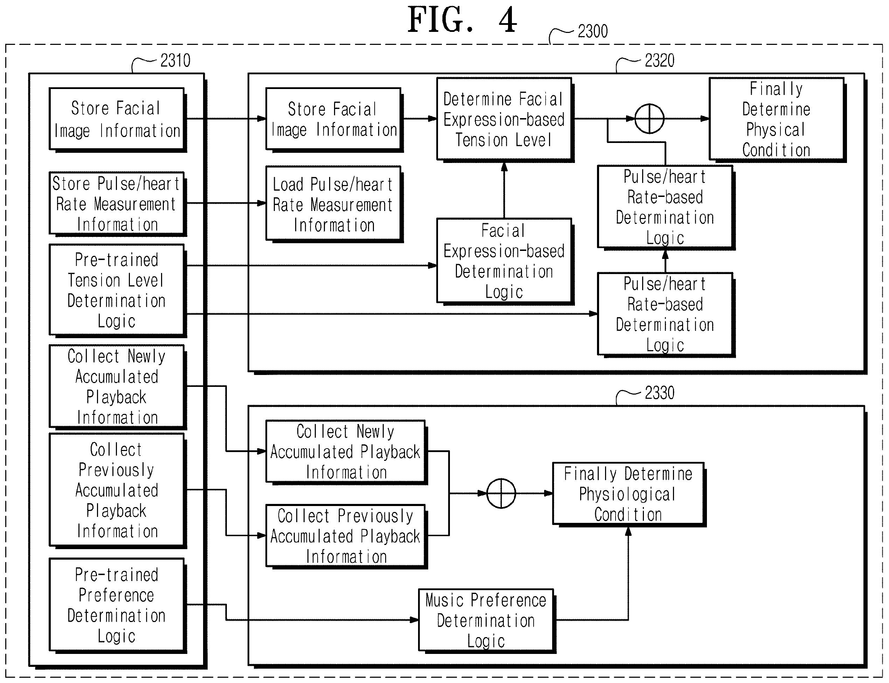

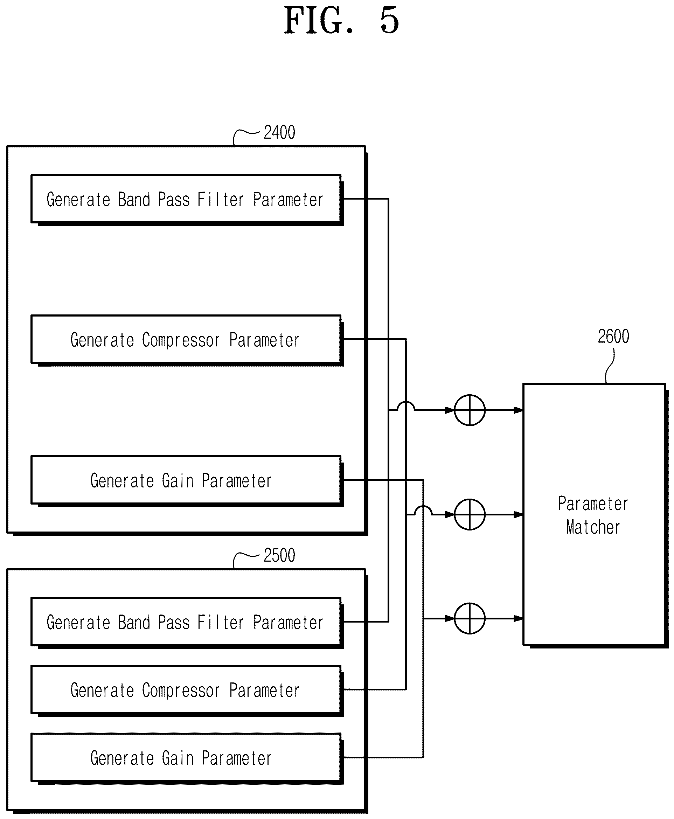

FIGS. 3 to 9 are diagrams for describing a vibro seat tuning system according to embodiments of the present disclosure.

Referring to FIGS. 3 to 5, the vibro seat tuning system may be composed of a server 2000 and a vibro seat 3000.

The server 2000 according to an embodiment of the present disclosure may include occupant biometric information storage 2100, occupant music preference storage 2200, a database 2300, a physical condition parameter generator 2400, a mental preference parameter generator 2500, and a parameter matcher 2600.

The server 2000 may be a vehicle customer relationship management (VCRM) server. The server 2000 may continuously receive vehicle input data.

In this regard, the vehicle input data may include image information received from an occupant information collector. The occupant information collector may collect occupant information from an in-cabin sensor or a camera disposed within a vehicle.

The vehicle input data may include steering wheel/seat belt information received from the occupant information collector. The occupant information collector may collect pulse/heart rate with a sensor mounted on a steering wheel, collect the heart rate with a sensor mounted on a seat belt, and calculate the pulse/heart rate by combining the collected information.

The vehicle input data may include information, obtained from a vehicle audio playback logger, in which music selections and genres preferences of each occupant are continuously accumulated after the sale of the vehicle.

In addition, the server 2000 may receive external input data. In this regard, the external input data may include streaming audio playback information, records, and the like obtained from the previous playback information database 2300.

The occupant biometric information storage 2100 according to an embodiment of the present disclosure may combine the image information with the pulse/heart rate based on the vehicle input data and store the combined information in the database 2300.

The occupant music preference storage 2200 according to an embodiment of the present disclosure may combine accumulated music selections with previous music selection information based on the vehicle input data and the external input data and store the combined information in the database 2300.

The database 2300 according to an embodiment of the present disclosure may be a VCRM database. Specifically, the database 2300 may include database storage 2310, a physical condition determiner 2320, and a mental preference determiner 2330.

The database storage 2310 according to an embodiment of the present disclosure may store facial image information and pulse/heart rate measurement information received from the occupant biometric information storage 2100. In addition, the database storage 2310 may store a pre-trained tension level determination logic.

Specifically, the tension level determination logic may include a facial expression-based determination logic and a pulse/heart rate-based determination logic. The facial expression-based determination logic and the pulse/heart rate-based determination logic may operate within the VCRM server. In this regard, data may be continuously accumulated in the VCRM server to perform data learning and real-time updating. Accordingly, after standardizing data of a plurality of general subjects to generate a comparison group, the standardized data may be compared with information of the occupant.

For example, the facial expression-based determination logic may be a tension level determination logic pre-trained through deep learning based on facial expression changes/facial temperature changes of an occupant in an image.

For example, the pulse/heart rate-based determination logic may be a tension level determination logic pre-trained based on the pulse and heart rate BPM of the occupant.

In addition, the database storage 2310 according to an embodiment of the present disclosure may store newly accumulated playback information collected from the vehicle audio playback logger. The database storage 2310 may store previously accumulated playback information received from the previous playback information DB. In addition, the database storage 2310 may store a pre-trained preference determination logic.

Specifically, the music preference determination logic may classify songs selected by a user based on genres based on accumulated information. For example, the genres may include ballad, hip-hop, jazz, and others.

The physical condition determiner 2320 according to an embodiment of the present disclosure may determine a physical condition of the occupant using occupant biometric information and occupant music preference information stored in the database storage 2310, together with a pre-trained body-based condition determination logic. To this end, the physical condition determiner 2320 may determine the physical condition of the occupant using a determination logic pre-trained through AI-based machine learning/deep learning.

Specifically, the physical condition determiner 2320 may load facial image information captured by the camera and stored in the database storage 2310. The physical condition determiner 2320 may determine a facial expression-based tension level of the occupant based on the facial expression-based determination logic pre-stored in the database storage 2310. In this regard, the physical condition determiner 2320 may quantify the tension level by comparing the facial expression-based determination logic with the facial image information.

For example, the tension level may be classified into levels 1 to 10 based on the degree of calmness and tension. Strong calmness may correspond to level 1, moderate calmness to level 2, slight calmness to level 3, very mild tension to level 4, slightly mild tension to level 5, mild tension to level 6, moderate tension to level 7, strong tension to level 8, fairly strong tension to level 9, and extreme tension to level 10.

In addition, the facial expression-based determination logic may analyze facial expression data in real time to determine tension state, fatigue, and mood change of the occupant. The facial expression-based determination logic may continuously improve an analysis model by reflecting new facial expression information in existing training data through real-time updating. The facial expression-based determination logic may automatically optimize analysis criteria by comparing general user data with global standard data using scaled-up data. For example, the facial expression-based determination logic may provide low-frequency vibrations for comfort in the case of a smiling facial expression, and may provide high-frequency blocking vibrations for relieving tension in the case of a tense facial expression.

Further, the physical condition determiner 2320 may load pulse/heart rate biometric information stored in the database storage 2310. The physical condition determiner 2320 may determine the pulse/heart rate-based tension level of the occupant based on a pulse/heart rate-based determination logic in the database storage 2310. In this regard, the physical condition determiner 2320 may quantify the tension level by comparing the pulse/heart rate-based determination logic with pulse/heart rate measurement information.

The pulse/heart rate-based determination logic may monitor pulse and heart rate change patterns in real time to evaluate the physical condition. The pulse/heart rate-based determination logic may operate in association with the VCRM server 2000 through real-time data updating and more accurately identify a current condition of a user through comparison with average data of other users.

The pulse/heart rate-based determination logic may analyze the scaled-up data to learn data of multiple users, thereby detecting an abnormal condition or providing feedback to maintain an optimal condition.

For example, when a heart rate rapidly increases, the pulse/heart rate-based determination logic may provide a sense of stability by reducing vibration intensity or changing a vibration pattern.

Further, the physical condition determiner 2320 may perform a weighted summation of the determined facial expression-based tension level and the determined pulse/heart rate-based tension level.

Thereafter, the physical condition determiner 2320 may determine a numerical value according to the weighted summation result to finally determine the physical condition.

The mental preference determiner 2330 according to an embodiment of the present disclosure may determine a mental preference of the occupant using a pre-trained psychological condition-based determination logic stored in the database storage 2310. To this end, the mental preference determiner 2330 may collect playlist information continuously accumulated in the vehicle, and determine the preference of the occupant using the determination logic pre-trained through AI-based machine learning/deep learning using the previous playback information stored in the database storage 2310.

Specifically, the mental preference determiner 2330 may load the newly accumulated playback information and the previously accumulated playback information. The mental preference determiner 2330 may perform a weight summation of the newly accumulated playback information and the previously accumulated playback information. In addition, the mental preference determiner 2330 may load the music preference determination logic.

The mental preference determiner 2330 may finally determine the mental preference based on the weighted summation result and the music preference determination logic.

The physical condition parameter generator 2400 according to an embodiment of the present disclosure may generate a band pass filter parameter. Specifically, the physical condition parameter generator 2400 may reset a low pass point of a filter as the pre-stored DB information according to the quantified tension level.

For example, when the physical condition corresponds to a tension level of 3.5, the physical condition parameter generator 2400 may set the band pass filter parameter to 1500Hz.

In addition, the physical condition parameter generator 2400 may generate a compressor parameter. Specifically, the physical condition parameter generator 2400 may reset in/out compressor application points of the filter as the pre-stored DB information according to the quantified tension level.

Finally, the physical condition parameter generator 2400 may adjust a final output gain value based on the pre-tuned DB.

The mental preference parameter generator 2500 according to an embodiment of the present disclosure may generate a band pass filter parameter.

Further, the mental preference parameter generator 2500 may generate a compressor parameter.

Finally, the mental preference parameter generator 2500 may adjust a final output gain value based on the pre-tuned DB.

The parameter matcher 2600 according to an embodiment of the present disclosure may receive a result of a weighted summation of the band pass filter parameter generated by the physical condition parameter generator 2400 and the band pass filter parameter generated by the mental preference parameter generator 2500.

In addition, the parameter matcher 2600 may receive a result of a weighted summation of the compressor parameter generated by the physical condition parameter generator 2400 and the compressor parameter generated by the mental preference parameter generator 2500.

In addition, the parameter matcher 2600 may receive a result of a weighted summation of the output gain value adjusted by the physical condition parameter generator 2400 and the output gain value adjusted by the mental preference parameter generator 2500.

Finally, the parameter matcher 2600 may apply a weight to match the parameters.

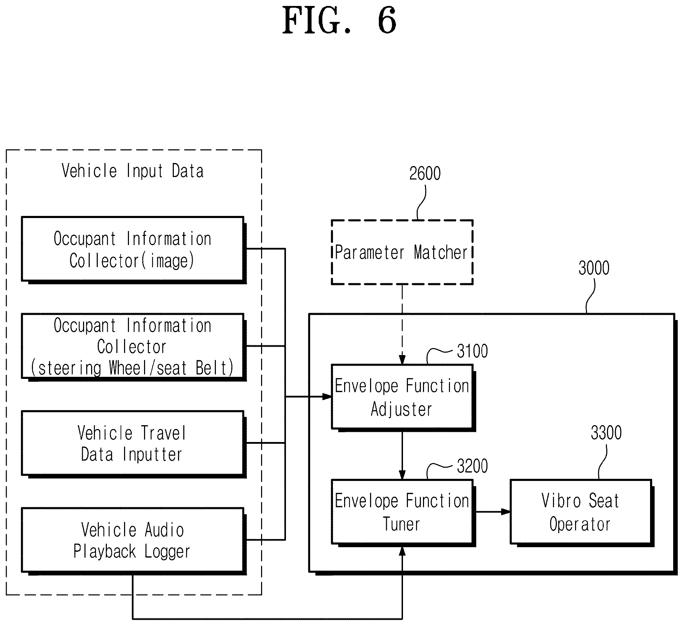

Referring to FIG. 6, the vibro seat 3000 according to an embodiment of the present disclosure may include an envelope function adjuster 3100, an envelope function tuner 3200, and a vibro seat operator 3300.

The envelope function adjuster 3100 according to the present disclosure may receive the image information and the steering wheel/seat belt information from the occupant information collector in real time.

In addition, the envelope function adjuster 3100 may receive, from the vehicle audio playback logger, the information in which the music selections and the genre preferences of each occupant are continuously accumulated after the sale of the vehicle.

In addition, the envelope function adjuster 3100 may receive a driving mode, a speed, a torque, an accelerator value, and the like from a vehicle travel data inputter. For example, the driving mode may include an eco mode, a normal/comfort mode, a sports mode, an off-road mode, a snow/sand mode, and the like.

The envelope function adjuster 3100 may re-adjust an envelope function based on vehicle travel data inputter information from band pass/compressor/gain information preset by the parameter matcher 2600.

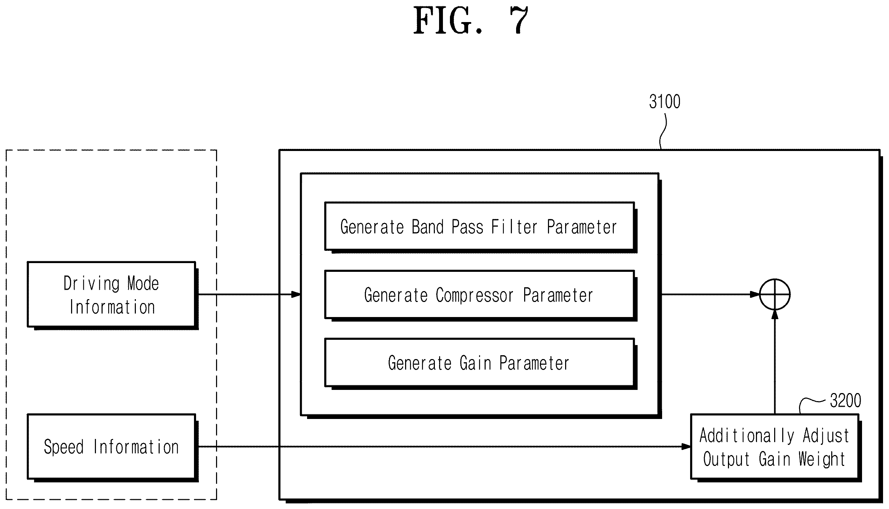

As illustrated in FIG. 7, the envelope function adjuster 3100 may readjust numerical values of the parameters according to each driving mode based on the driving mode information received from the vehicle travel data inputter. Specifically, the envelope function adjuster 3100 may adjust the band pass filter parameter, the compressor parameter, and the gain parameter.

In addition, the envelope function adjuster 3100 may additionally adjust an output gain weight for each bandwidth based on vehicle speed information.

The envelope function adjuster 3100 may transmit a result of a weighted summation of the readjusted parameter value and gain weight to the envelope function tuner 3200.

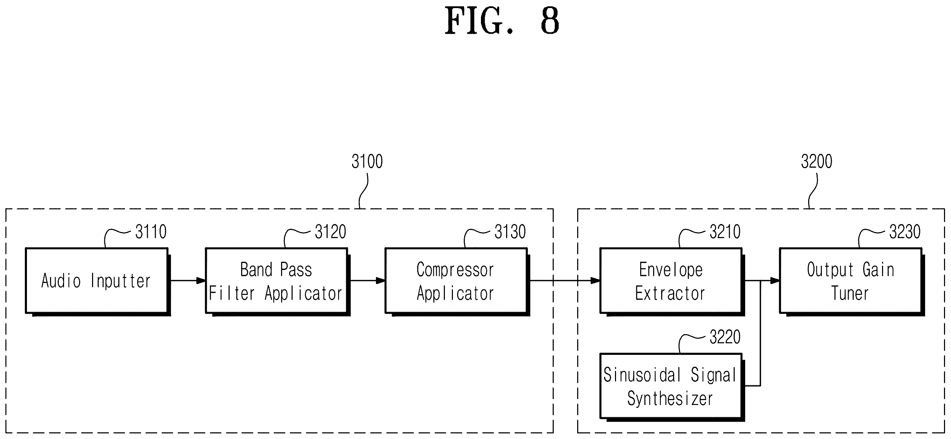

Referring to FIG. 8, the envelope function adjuster 3100 includes an audio inputter 3110, a band pass filter applicator 3120, and a compressor applicator 3130, and the envelope function tuner 3200 includes an envelope extractor 3210, a sinusoidal signal synthesizer 3220, and an output gain tuner 3230.

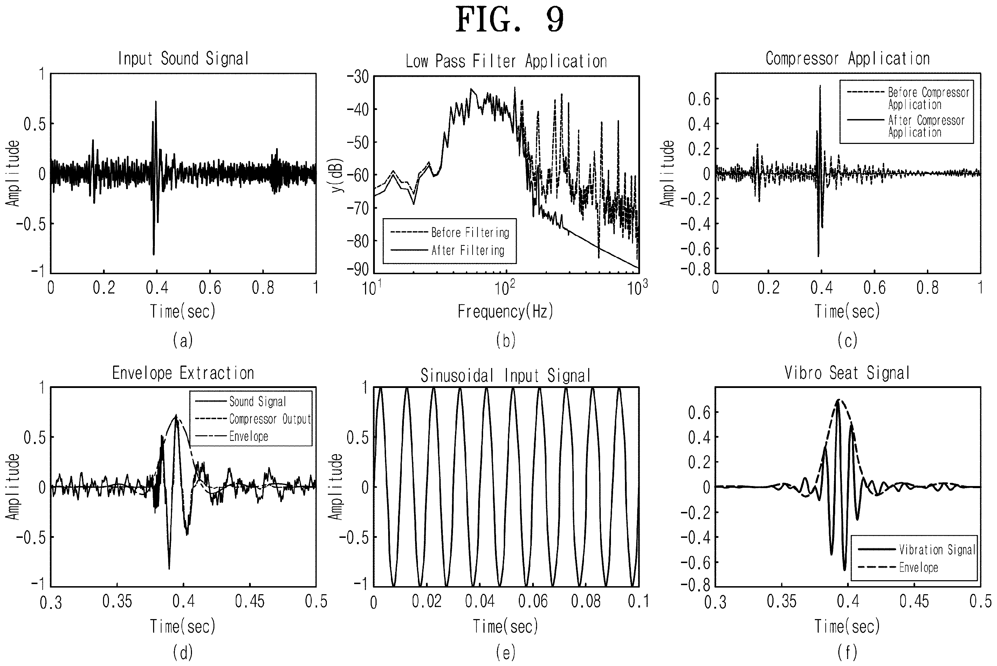

As illustrated in (a) in FIG. 9, the audio inputter 3110 of the envelope function adjuster 3100 may collect an input sound signal within the vehicle using a microphone.

As illustrated in (b) in FIG. 9, the band pass filter applicator 3120 may convert a time domain of the input sound signal to a frequency domain and then attenuate signal components in specific bandwidths using the band pass filter. For example, the band pass filter may be a low pass filter.

As illustrated in (c) in FIG. 9, the compressor applicator 3130 may convert a frequency domain of the signal to which the band pass filter has been applied to a time domain and then reduce a signal level using a compressor.

As illustrated in (d) in FIG. 9, the envelope extractor 3210 of the envelope function tuner 3200 may extract an envelope of the signal using the low pass filter.

As illustrated in (e) in FIG. 9, the sinusoidal signal synthesizer 3220 may generate a sinusoidal (Sin) wave having a predetermined period.

As illustrated in (f) in FIG. 9, the output gain tuner 3230 may perform a convolution on the envelope signal, extracted by the envelope extractor 3140, and the sinusoidal wave to generate a vibro seat signal.

Accordingly, the envelope function adjuster 3100 according to an embodiment of the present disclosure may apply a tuning parameter adjusted by the server, and the envelope function tuner 3200 may perform tuning using an envelope function.

In this regard, the envelope function tuner 3200 according to an embodiment of the present disclosure may apply information processed by the VCRM server in the form of parameters by being involved in band pass filtering/compression/low pass filtering/sinusoidal signal generation.

In addition, the vibro seat operator 3300 according to an embodiment of the present disclosure may operate the vibro seat based on the vibro seat signal generated by the envelope function tuner 3200.

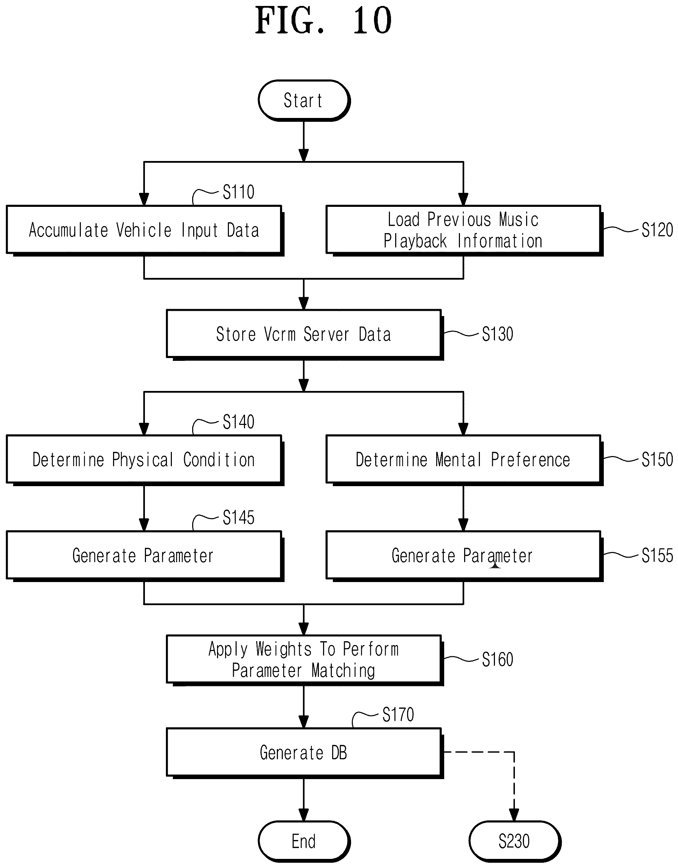

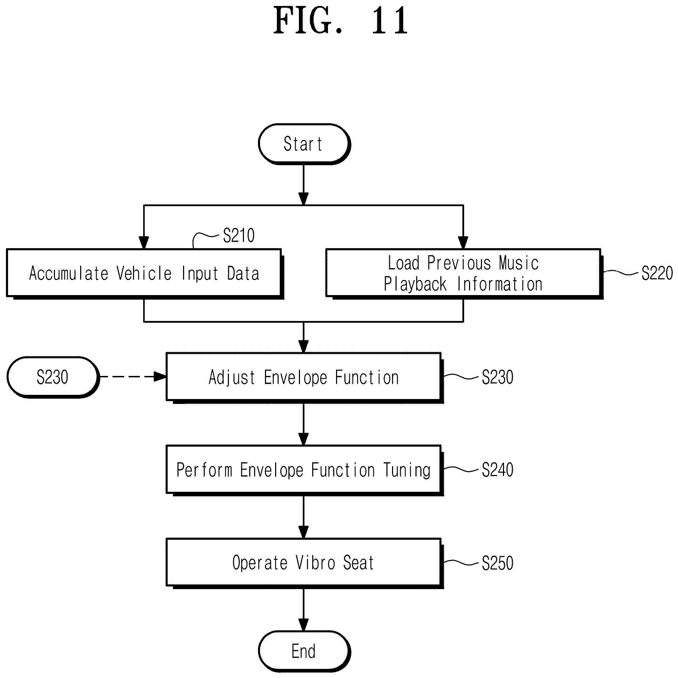

FIGS. 10 and 11 are flowcharts for describing a method for tuning a vibro seat according to embodiments of the present disclosure.

Referring to FIGS. 10 and 11, the server 2000 of the vibro seat tuning system may receive accumulated vehicle input data (S110).

The server 2000 may load the previous music playback information (S120).

The server 2000 may store VCRM server data such as the accumulated vehicle input data and the loaded previous music playback information (S130). For example, the server 2000 may generate the VCRM data based on the occupant biometric information and the occupant music preference information.

The server 2000 may determine a physical condition based on the VCRM server data (S140). To this end, the server 2000 may determine the physical condition based on the occupant biometric information. Specifically, the server 2000 may determine the facial expression-based tension level by comparing the facial image information of the occupant with the facial expression-based determination logic, determine the pulse/heart rate-based tension level by comparing the pulse information and the heart rate information of the occupant with the pulse/heart rate-based determination logic, and determine the physical condition by performing a weighted summation of the facial expression-based tension level and the pulse/heart rate-based tension level.

The server 2000 may generate a physical condition parameter based on the determined physical condition information (S145).

Further, the server 2000 may generate the physical condition parameter based on the determined physical condition. Specifically, the server 2000 may generate a first band pass filter parameter, generate a first compressor parameter, and generate a first output gain parameter.

Further, the server 2000 may determine a mental preference based on the VCRM server data (S150).

In addition, the server 2000 may determine the mental preference based on the occupant music preference information. Specifically, the server 2000 may collect new playback information of the occupant accumulated within the vehicle, collect the pre-stored previous playback information of the occupant, and compare the new playback information and the previous playback information with the music preference determination logic to determine the mental preference.

The server 2000 may generate a mental preference parameter based on the determined mental preference information (S155).

The server 2000 may generate the mental preference parameter based on the determined mental preference. Specifically, the server 200 may generate a second band pass filter parameter, generate a second compressor parameter, and generate a second output gain parameter.

The server 2000 may apply weights to the generated physical condition parameter and mental preference parameter to perform parameter matching (S160).

Specifically, the server 2000 may generate a third band pass filter parameter by performing a weighted summation of the first band pass filter parameter and the second band pass filter parameter, generate a third compressor parameter by performing a weighted summation of the first compressor parameter and the second compressor parameter, and generate a third output gain filter parameter by performing a weighted summation of the first output gain parameter and the second output gain parameter.

The server 2000 may generate a vibro seat tuning parameter by matching the physical condition parameter with the mental preference parameter.

The server 2000 may generate the database based on the parameter matching result (S170).

The vibro seat 3000 of the vibro seat tuning system may receive the accumulated vehicle input data in real time (S210).

The vibro seat 3000 may load the previous music playback information (S220).

The vibro seat 3000 may adjust the envelope function based on the accumulated vehicle input data received in real time, the loaded previous music playback information, and the parameter matching result received from the server 2000 (S230). Specifically, the vibro seat 3000 may receive audio output in real time within the vehicle, apply the band pass filter configured with the third band pass filter parameter to the received audio, and apply the compressor configured with the third compressor parameter to the audio to which the band pass filter has been applied.

The vibro seat 3000 may perform envelope function tuning based on the envelope function adjustment result (S240). Specifically, the vibro seat 3000 may extract the envelope by applying the low pass filter to the audio to which the compressor has been applied, generate a sinusoidal signal, and generate a vibro seat signal by performing a convolution between the extracted envelope and the generated sinusoidal wave.

The vibro seat 3000 may operate based on the envelope function tuning result (S250). Specifically, the vibro seat 3000 may operate based on the generated vibro seat signal.

That is, the technical idea of the present disclosure is applicable to an entire autonomous vehicle or is also applicable to only some components within the autonomous vehicle. The scope of the present disclosure should be determined according to the matters described in the claims.

In another aspect of the present disclosure, the above-described proposal or operation of the present disclosure may be provided as a code that may be implemented, performed, or executed by a "computer" (a comprehensive concept including a system on chip (SoC), a microprocessor, or the like) or an application, a computer-readable storage medium, a computer program product, or the like, which stores or contains the code. Further, such provision also falls within the scope of the present disclosure.

The detailed descriptions of the preferred embodiments of the present disclosure disclosed as above are provided to allow those skilled in the art to implement and embody the present disclosure. While the present disclosure has been described and illustrated herein with reference to the preferred embodiments thereof, those skilled in the art will understand that the present disclosure may be variously modified and changed without departing from the scope of the present disclosure. For example, those skilled in the art may use each of the components described in the above-described embodiments in a manner of combining them with each other.

Accordingly, the present disclosure is not intended to be limited to the embodiments described herein, but is intended to give the broadest scope consistent with the principles and novel features disclosed herein.

## Claims

1. A method for tuning a vibro seat, the method comprising:
generating vehicle customer relationship management, VCRM, data based on biometric information of an occupant and music preference information of the occupant;
generating a vibro seat tuning parameter based on the generated VCRM data;
adjusting and tuning an envelope function based on the occupant biometric information and the occupant music preference information received in real time and the vibro seat tuning parameter; and
operating the vibro seat based on the tuned envelope function.

2. The method of claim 1, wherein the generating of the VCRM data includes:
determining a physical condition based on the occupant biometric information;
generating a physical condition parameter based on the determined physical condition;
determining a mental preference based on the occupant music preference information;
generating a mental preference parameter based on the determined mental preference; and
generating the vibro seat tuning parameter by matching the physical condition parameter and the mental preference parameter with each other.

3. The method of claim 2, wherein the determining of the physical condition based on the occupant biometric information includes:
determining a facial expression-based tension level by comparing facial image information of the occupant with a facial expression-based determination logic;
determining a pulse/heart rate-based tension level by comparing pulse information and heart rate information of the occupant with a pulse/heart rate-based determination logic; and
determining the physical condition by performing a weighted summation of the facial expression-based tension level and the pulse/heart rate-based tension level.

4. The method of claim 3, wherein the generating of the physical condition parameter based on the determined physical condition includes:
generating a first band pass filter parameter;
generating a first compressor parameter; and
generating a first output gain parameter.

5. The method of claim 4, wherein the determining of the mental preference based on the occupant music preference information includes:
collecting new playback information of the occupant accumulated within a vehicle;
collecting pre-stored previous playback information of the occupant; and
determining the mental preference by comparing the new playback information and the previous playback information with a music preference determination logic.

6. The method of claim 5, wherein the generating of the mental preference parameter based on the determined mental preference includes:
generating a second band pass filter parameter;
generating a second compressor parameter; and
generating a second output gain parameter.

7. The method of claim 6, wherein the generating of the vibro seat tuning parameter by matching the physical condition parameter and the mental preference parameter with each other includes:
generating a third band pass filter parameter by performing a weighted summation of the first band pass filter parameter and the second band pass filter parameter;
generating a third compressor parameter by performing a weighted summation of the first compressor parameter and the second compressor parameter; and
generating a third output gain filter parameter by performing a weighted summation of the first output gain parameter and the second output gain parameter.

8. The method of claim 7, wherein the adjusting and tuning of the envelope function includes:
receiving audio output in real time within the vehicle;
applying a band pass filter configured with the third band pass filter parameter to the received audio;
applying a compressor configured with the third compressor parameter to the audio to which the band pass filter has been applied;
extracting an envelope by applying a low pass filter to the audio to which the compressor has been applied;
generating a sinusoidal signal; and
generating a vibro seat signal by performing a convolution on the extracted envelope and the generated sinusoidal signal.

9. The method of claim 8, wherein the adjusting and tuning of the envelope function further includes:
receiving vehicle travel data including driving mode information and speed information;
adjusting the vibro seat tuning parameter based on the driving mode information; and
adjusting an output gain weight based on the speed information.

10. A system for turning a vibro seat, the system comprising:
a server configured to:
generate VCRM data based on biometric information of an occupant and music preference information of the occupant; and
generate a vibro seat tuning parameter based on the generated VCRM data; and the vibro seat configured to:
adjust and tune an envelope function based on the occupant biometric information and the occupant music preference information received in real time and the vibro seat tuning parameter; and
operate based on the tuned envelope function.
